# FASCICULE DE BREVET EUROPEEN

(11) **EP 0 591 048 B1**
(45) Date de publication et mention de la délivrance du brevet: **16.06.1999**
(21) Numéro de dépôt: 93402375.5
(22) Date de dépôt: 29.09.1993
(51) Int. Cl.: A24D 3/06, A24D 3/14, A61K 31/185

(54) **Filtre à cigarette pour l'administration de taurine par inhalation**
Zigarettenfilter für inhalatorische Anwendung von Taurin
Cigarette filter for the administration of taurine by inhalation

(30) Priorité: 30.09.1992 FR 9211645
(43) Date de publication de la demande: 06.04.1994
(73) Titulaire: Covarrubias, Jesus, Mexico 20-DF (MX)
(72) Inventeur: Covarrubias, Jesus, Mexico 20-DF (MX)
(74) Mandataire: L'Helgoualch, Jean

(56) Documents cités:
- EP-A- 0 003 064
- EP-A- 0 058 463
- WO-A-92/17170
- FR-A- 2 167 382
- GB-A- 1 204 018
- Am. Rev. Resp. Dis., vol.131, n 4 suppl., 1985. Abstract A179, Gordon & al.: 'Taurine Prevents Nitrogen Dioxide Disruption of Bronciolar Epithelial Barrier Function'. & 80th Annual Meeting of the American Thoracic Society, Anaheim, CA, USA, 12-15 May 1985

## Description

La présente invention concerne un dispositif pour l'administration de substances médicamenteuses ou nutritionnelles par voie orale, et plus particulièrement un dispositif destiné à l'administration par inhalation d'une substance pharmacologiquement active telle que la taurine au niveau des poumons d'un fumeur, notamment dans le cadre d'un traitement préventif des affections des voies respiratoires.

On connaît divers dispositifs selon lesquels on intègre des substances actives ou aromatiques dans des cigarettes afin d'en modifier le goût ou de leur conférer des propriétés additionnelles. Par exemple, les brevets US-A-3.991.773 et US-A-3.635.226 décrivent des dispositifs pour humidifier la fumée d'une cigarette en incorporant des capsules ou micro-capsules frangibles remplies d'un liquide, dans le filtre de la cigarette. Le fumeur peut provoquer l'humidification en pressant le filtre pour rompre les capsules, ou au contraire choisir de laisser les capsules intactes pour aspirer de la fumée sèche. Le liquide incorporé dans les capsules peut contenir par exemple des arômes ou une solution de salive synthétique.

On sait aussi incorporer dans le tabac de la cigarette une substance possédant des propriétés médicinales. Le brevet US-A-4.498.495 décrit l'incorporation d'un interféron dans le tabac d'une cigarette en vue de l'administrer directement aux poumons du fumeur. Le brevet FR-A-2.167.382 décrit l'incorporation d'hormones dans le tabac ou dans le filtre d'une cigarette. Les brevets GB-A-1.204.018 et EP-A-003.064 décrivent des filtres de cigarette comprenant des capsules contenant de la vitamine A, susceptibles de se rompre sous l'effet de la chaleur.

La taurine (acide 2-aminoéthane sulfonique) est un acide aminé de faible toxicité possédant quelques propriétés pharmacologiques connues permettant d'envisager son application en thérapeutique pour le traitement de l'insuffisance cardiaque mais son activité est trop faible pour être utilisable en pratique comme médicament. Des amides de taurine présentant des propriétés mucolytiques, hépatoprotectrices, détoxifiantes et normolipémiantes, sont décrits dans le brevet EP-A-307.788.

R.E. Gordon et al. dans un article intitulé "Taurine Protects Hamster Bronchioles From Acute NO₂-Induced Alterations", AJP (déc. 1986), décrit l'utilisation de la taurine dans l'alimentation de hamsters, procurant une amélioration de la résistance aux effets nocifs du dioxyde d'azote NO₂ que l'on trouve dans la pollution urbaine et qui est un composant de la fumée du tabac. Selon une hypothèse présentée dans cet article, la taurine serait distribuée dans les tissus de l'animal et participerait à diverses réactions biochimiques ayant pour effet de stabiliser les membranes, de piéger les radicaux libres et d'éviter une peroxydation. L'effet protecteur de la taurine sur les membranes et contre des composés toxiques tels que des oxydants, est aussi décrit par G.E. Gaull dans "Taurine as a Conditionally Essential Nutrient in Man", J. of Am. College of Nutrition 5:121-125 (1986) qui envisage un tel effet chez l'homme.

Plus récemment, l'administration de taurine par voie intraveineuse à des patients qui ont subi une intervention chirurgicale de pontage coronarien, a fait apparaître une diminution de la dégradation myocardiaque en produisant un effet anti-oxydant et piégeant ou séquestrant. On peut se référer à Ferreira et al. "Reduction of Reperfusion Injury During Myocardial Revascularization with Taurine Bolus" Université de Milan, 15-20 juillet 1990.

On a également montré que l'administration de diméthylsulfoxyde sous forme nébulisée, par inhalation, est efficace pour le traitement d'affections pulmonaires induites par la fumée. On peut se référer à Kimura et al. dans "Treatment of Smoke-Induced Pulmonary Injury by Nebulized Dimethylsulfoxide", Alan R. Riss, Inc. (1988). Ces résultats sont fondés sur des expériences effectuées sur des animaux.

D'autres résultats d'expérimentations publiés ont établi l'utilité de la taurine pour procurer un effet anti-mutagène par inhibition de divers agents supposés être carcinogènes, qui peuvent provoquer des modifications de l'ADN. Ces agents contiennent des radicaux libres réactifs. On peut se référer à Laidlaw et al. dans "Anti-Mutagenic Effects of Taurine - A Bacterial Assay System", Cancer Research, 49, 6600-6604 (Déc. 1989).

La présente invention a pour objet un dispositif pour l'administration de taurine à des fumeurs, par inhalation.

Plus particulièrement, l'invention a pour objet un filtre de cigarette comprenant un matériau filtrant destiné à filtrer la fumée résultant de la combustion du tabac qui traverse ledit matériau filtrant, et des moyens pour incorporer la taurine dans ledit matériau filtrant et l'introduire dans la fumée quand elle traverse ledit matériau filtrant.

La présente invention concerne aussi un procédé pour administrer de la taurine jusqu'aux poumons d'un fumeur, consistant à incorporer la taurine dans un filtre de cigarette sous forme de poudre ou de solution. enfin, l'invention a pour objet l'utilisation de la taurine dans la fabrication d'un médicament pour la prévention des effets nocifs de la fumée de combustion du tabac, notamment chez les fumeurs.

Suivant une forme préférentielle de réalisation, le dispositif suivant la présente invention est constitué par un filtre de cigarette comprenant :
- un matériau filtrant assurant la filtration de la fumée émise par le tabac en combustion, lorsque cette fumée traverse ledit matériau filtrant;
- au moins une capsule frangible contenant de l'eau ou une solution aqueuse, placée dans ledit matériau filtrant, de telle sorte qu'une pression exercée sur le filtre provoque la rupture de la capsule et la libération de l'eau ou de la solution dans le matériau filtrant.

La taurine peut être soit sous forme de poudre incorporée dans le matériau filtrant qui contient en outre au moins une capsule contenant de l'eau ou une solution aqueuse, soit sous forme de solution contenue dans la ou les capsules incorporées dans le matériau filtrant. Lorsque la taurine est sous forme de poudre, elle est de préférence associée à des microsphères contenant de l'eau, réparties dans le matériau filtrant.

Les caractéristiques de l'invention apparaîtront plus en détail dans la description suivante, relative à une forme préférentielle de réalisation, en référence aux dessins annexés, qui représentent :
- Figure 1 :: une vue en coupe longitudinale du bout filtre d'une cigarette comprenant le dispositif de l'invention.
- Figure 2 :: une vue en coupe d'un filtre montrant une deuxième forme de réalisation de l'invention.
- Figure 3 :: une vue en coupe d'un filtre montrant une troisième forme de réalisation de l'invention.

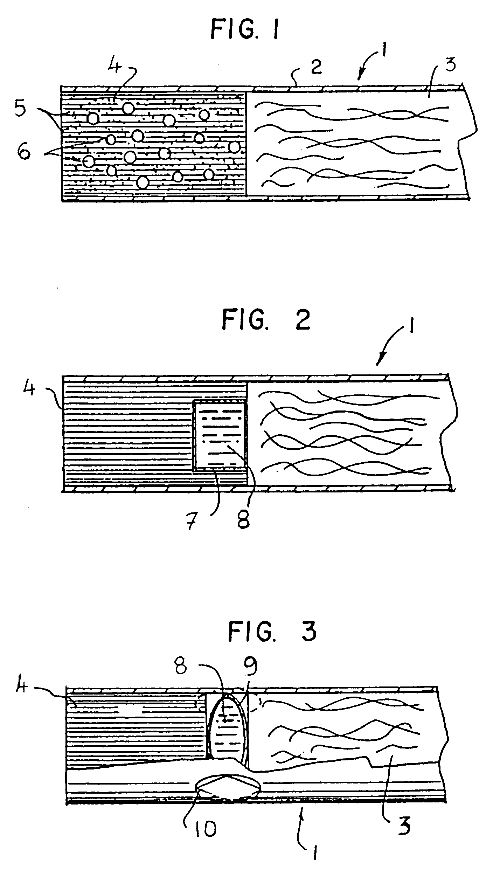

Comme le montre la figure 1, le dispositif suivant l'invention comprend une bout filtre (1) de cigarette comprenant un papier (2) constituant une enveloppe contenant du tabac (3) et un filtre constitué par un matériau de filtration (4), tel que de la cellulose compactée ou des fibres similaires.

Suivant la présente invention, les moyens pour incorporer la taurine dans le matériau filtrant sont prévus sous la forme d'une poudre de taurine (5) finement divisée, qui est répartie uniformément dans tout le matériau filtrant (4). La poudre, qui doit avoir la consistance d'une poudre légère de talc, est automatiquement entraînée dans la fumée lorsque celle-ci, provenant du tabac (3) en combustion, traverse le matériau filtrant (4) et parvient jusqu'aux poumons du fumeur.

Si nécessaire, suivant l'invention, la taurine peut être mise en solution aqueuse répartie dans le matériau filtrant (4) en pressant le filtre (1) entre les doigts pour provoquer l'écrasement et la rupture des parois de microsphères (6) contenant de l'eau distillée, une solution saline physiologique ou une solution aqueuse non toxique. Ainsi l'écrasement des microsphères par une pression douce sur le filtre en le faisant rouler entre les doigts, provoque la rupture des parois minces faites par exemple en résine époxy ou en toute autre substance inerte, et la solution aqueuse ou l'eau qui y est contenue se répand alors dans tout le matériau filtrant (4) et forme une solution avec la poudre de taurine, la taurine étant soluble dans l'eau.

La présence d'une solution de taurine dans le matériau filtrant traversé par une fumée en mouvement relativement rapide, et qui est tiède, facilite une certaine évaporation et une certaine atomisation de la solution qui se trouve alors entraînée jusque dans les poumons du fumeur.

Les figures 2 et 3 représentent deux autres formes de réalisation de la présente invention, où les mêmes références sont utilisées pour identifier les mêmes composants que sur la figure 1.

La figure 2 montre une capsule (7) frangible à paroi étanche contenant une solution de taurine (8), placée au milieu du matériau filtrant (4) à proximité de l'interface avec le tabac. Une pression exercée sur le filtre à ce niveau provoque l'écrasement de la capsule, la rupture des parois et la libération de la solution de taurine dans le matériau filtrant.

La figure 3 représente une troisième forme de réalisation du filtre suivant la présente invention, où une capsule de forme oblongue (9) est placée dans l'espace, délimité par le papier (2), séparant le tabac (3) du matériau filtrant (4). Des marques ou repères (10) sont formés sur la surface extérieure du papier (2) de la cigarette de telle sorte que le fumeur puisse repérer l'endroit où il doit presser la cigarette pour provoquer l'écrasement de la capsule (9) et l'éclatement de ses parois. Bien que la plus grande partie de la solution de taurine (8) s'infiltre dans le matériau filtrant (4) et dans le tabac (3) se trouvant immédiatement à proximité, une petite partie de la solution reste dans l'espace séparant le tabac (3) du matériau filtrant (4) et sert ainsi à l'humidification, ainsi qu'à l'introduction de la solution de taurine dans la fumée qui traverse le filtre.

Les réalisations représentées sur les figures 2 et 3 peuvent aussi être modifiées en plaçant de l'eau distillée ou une autre solution aqueuse dans les capsules (7) et (9), la taurine en poudre étant placée dans le matériau filtrant (4). Suivant l'invention, le filtre peut aussi être du type de celui de la figure 1 mais ne contenant pas les sphères frangibles et contenant seulement la taurine en poudre.

Un avantage particulier résultant de la présence de taurine, soit sous forme de poudre, soit sous forme de solution, dans le filtre, est lié à la température de décomposition de la taurine qui est de 300°C. L'introduction de taurine au milieu de la charge de tabac détruirait manifestement l'effet recherché dans l'invention car la taurine serait décomposée au fur et à mesure que la combustion progresserait vers le filtre. L'invention diffère ainsi du brevet US-A-4.498.485 qui propose l'inclusion d'une substance pharmacologiquement active, l'interféron, dans le tabac de la cigarette.

La présence de taurine, même en faible quantité, dans la fumée d'une cigarette, procure un système d'administration efficace de la taurine.

En effet, la taurine, lorsqu'elle est administrée aux poumons, procure un effet anti-oxydant avantageux qui est connu, comme indiqué ci-dessus. Cet effet se trouve potentialisé lorsque cette administration se fait en présence de fumée de combustion de cigarette présentant un effet oxydant. Bien que l'activité de la taurine soit supposée s'exercer principalement dans les poumons, elle peut aussi agir directement sur la fumée par une sorte d'effet tampon avant que celle-ci n'atteigne les poumons du fumeur.

D'autre part, l'administration de la taurine par inhalation, par le dispositif conforme à la présente invention, diffère sensiblement de l'administration par voie orale, notamment comme additif nutritionnel, et ne procure pas les mêmes effets. Dans le cas de l'administration classique par voie orale, la taurine se trouve exposée au milieu acide dans l'estomac et est absorbée dans la tractus gastro-intestinal. Au contraire, l'invention permet de mettre directement la taurine en contact avec la paroi interne des poumons. Comme indiqué ci-dessus, l'effet de la taurine se trouve potentialisé, même pour les quantités de taurine relativement faibles incorporées dans le filtre de cigarette.

On constate que l'administration de taurine par le dispositif de l'invention procure un effet protecteur contre les effets nocifs de la fumée de combustion du tabac, vérifié par le test des bronchospasmes induits par la nébulisation d'eau distillée par ultrasons. On constate une diminution des bronchospasmes quand la taurine est administrée régulièrement par le dispositif de l'invention. Ceci montre que l'administration régulière de taurine par inhalation, par le dispositif suivant la présente invention, exerce un effet préventif non seulement contre les effets nocifs de la combustion du tabac, mais aussi contre certaines affections des voies respiratoires comme l'asthme et la bronchite chronique.

La solution de taurine présente dans les capsules du dispositif des figures 2 et 3 est de préférence une solution dont la teneur en taurine est de 0,2 à 2,0% en poids. Les microsphères d'eau du dispositif de la figure 1 doivent être incorporées dans le matériau filtrant en une quantité telle que la solution de taurine formée ait une concentration en taurine d'environ 100mg/ml. La quantité totale de taurine présente dans le filtre est généralement comprise entre 25 mg et 200 mg, de préférence entre 50 mg et 120 mg.

## Revendications

1. Dispositif pour l'administration de taurine par inhalation constitué par un filtre de cigarette caractérisé en ce qu'il comprend :
- un matériau filtrant destiné à filtrer la fumée résultant de la combustion du tabac qui traverse ledit matériau filtrant, et
- des moyens pour incorporer la taurine dans ledit matériau filtrant et l'introduire dans la fumée quand elle traverse ledit matériau filtrant.

2. Dispositif selon la revendication 1, caractérisé en ce qu'il comprend :
- un matériau filtrant assurant la filtration de la fumée;
- au moins une capsule frangible contenant de l'eau ou une solution aqueuse, placée dans ledit matériau filtrant, de telle sorte qu'une pression exercée sur le filtre provoque la rupture de la capsule et la libération de l'eau, ou de la solution, dans le matériau filtrant.

3. Dispositif selon l'une quelconque des revendications 1 et 2, caractérisé en ce que le matériau filtrant contient des microsphères frangibles contenant de l'eau ou une solution aqueuse.

4. Dispositif selon l'une quelconque des revendications 1 à 3, caractérisé en ce que la taurine est sous forme de poudre incorporée dans le matériau filtrant.

5. Dispositif selon l'une quelconque des revendications 1 à 3, caractérisé en ce que la taurine est sous forme de solution contenue dans au moins une capsule frangible incorporée dans le matériau filtrant.

6. Utilisation de la taurine pour la fabrication d'un médicament pour administration par inhalation pour la prévention des effets nocifs de la fumée de combustion du tabac.

## Claims

1. A device for administering taurine by inhalation, consisting of a cigarette filter characterized in that it comprises :
- a filtration material for filtering the smoke from burning tobacco which passes through said filtration material, and
- means for including taurine into said filtration material and introducing it into the smoke as it passes through said filtration material.

2. Device according to claim 1, characterized in that it comprises :
- a filtration material for filtering smoke;
- at least one rupturable capsule containing water or an aqueous solution, located in said filtration material, so that the capsule is ruptured by a pressure on the filter, and the water or the aqueous solution is distributed into the filtration material.

3. Device according to any of claims 1 and 2, characterized in that said filtration material contains rupturable microspheres containing water or an aqueous solution.

4. Device according to any of claims 1 to 3, characterized in that taurine is in the form of a powder introduced into the filtration material.

5. Device according to any of claims 1 to 3, characterized in that taurine is in the form of a solution contained in at least one rupturable capsule introduced into the filtration material.

6. The use of taurine for the manufacture of a drug for administration by inhalation for protection against the harmful effects of the smoke from the burning tobacco.

## Patentansprüche

1. Vorrichtung zur Verabreichung von Taurin durch Inhalation, bestehend aus einem Zigarettenfilter, dadurch gekennzeichnet, daß sie folgendes umfaßt:
- Filtermaterial, das dazu bestimmt ist, den aus der Verbrennung von Tabak resultierenden Rauch zu filtern, der durch das Filtermaterial hindurchgeht, und
- Mittel zum Aufnehmen des Taurins in das Filtermaterial und Einbringen desselben in den Rauch, wenn er durch das Filtermaterial hindurchgeht.

2. Vorrichtung nach Anspruch 1, dadurch gekennzeichnet, daß sie folgendes umfaßt:
- Filtermaterial, das die Filterung des Rauchs gewährleistet;
- zumindest eine zerbrechliche Kapsel, die Wasser oder eine wäßrige Lösung enthält und so im Filtermaterial angeordnet ist, daß auf den Filter ausgeübter Druck ein Bersten der Kapsel und Freisetzung des Wassers oder der Lösung in das Filtermaterial bewirkt.

3. Vorrichtung nach einem der Ansprüche 1 oder 2, dadurch gekennzeichnet, daß das Filtermaterial zerbrechliche Mikrokügelchen enthält, die Wasser oder eine wäßrige Lösung enthalten.

4. Vorrichtung nach einem der Ansprüche 1 bis 3, dadurch gekennzeichnet, daß das Taurin in Form von Pulver vorliegt, das im Filtermaterial eingeschlossen ist.

5. Vorrichtung nach einem der Ansprüche 1 bis 3, dadurch gekennzeichnet, daß das Taurin in Form einer Lösung vorliegt, die in zumindest einer zerbrechlichen Kapsel enthalten ist, die im Filtermaterial eingeschlossen ist.

6. Verwendung von Taurin zur Herstellung eines Medikaments zur Verabreichung durch Inhalation zur Vorbeugung gegen die schädlichen Wirkungen von Rauch aus der Verbrennung von Tabak.
